# EUROPÄISCHE PATENTANMELDUNG

(11) **EP 1 728 524 A1**
(43) Veröffentlichungstag der Anmeldung: **06.12.2006**
(21) Anmeldenummer: 06010172.2
(22) Anmeldetag: 17.05.2006
(51) Int. Cl.: A61L 9/12, A01M 1/20, B32B 27/08, B32B 7/00, B60H 3/00, B65D 77/20

(54) **Vorrichtung zur Freisetzung von Wirkstoffen**

(30) Priorität: 03.06.2005 DE 102005026020
(71) Anmelder: TUFTY GmbH, 67661 Kaiserlauten (DE)
(72) Erfinder: Karg, Jörn E., 67659 Kaiserslautern (DE)
(74) Vertreter: Beckord, Klaus

(57) **Zusammenfassung**

Die vorliegende Erfindung betrifft eine Vorrichtung zur Freisetzung wenigstens eines Wirkstoffes und ein Verfahren zur Herstellung derselben. Des Weiteren betrifft die vorliegende Erfindung die Verwendung der Vorrichtung zur Desodorierung und/oder Beduftung von Räumen, zur Abwehr und/oder Bekämpfung von Insekten und zur Behandlung von Erkrankungen der Atemwege sowie von Kopfschmerzen und Schlafstörungen.

## Beschreibung

Die vorliegende Erfindung betrifft eine Vorrichtung zur Freisetzung wenigstens eines Wirkstoffes und ein Verfahren zur Herstellung derselben. Des Weiteren betrifft die vorliegende Erfindung die Verwendung der Vorrichtung zur Desodorierung und/oder Beduftung von Räumen, zur Abwehr und/oder Bekämpfung von Insekten und zur Behandlung von Erkrankungen der Atemwege sowie von Kopfschmerzen und Schlafstörungen.

Vorrichtungen zur Freisetzung von Wirkstoffen sind in den verschiedensten Bereichen einsetzbar. So eignen sich solche Vorrichtungen beispielsweise zur Desodorierung und/oder Beduftung von Räumen, wie beispielsweise Innenräume von Gebäuden, Fahrzeugen, Geschirrspülmaschinen, Waschmaschinen, Wäschetrocknern, Kühlschränken, Mülleimern, Katzentoiletten und Einrichtungsgegenständen, wie Kleiderschränke, Vorratsschränke, etc. Dufthaltige Artikel zur Desodorierung und/oder Beduftung von Geschirrspülmaschinen und Wäschetrocknern sind beispielsweise in den internationalen Veröffentlichungen WO 91/00744 und WO 85/00981, in der deutschen Patentschrift DE 42 05 975 C2 sowie in den deutschen Veröffentlichungen DE 102 37 066 A1 und DE 103 03 352 A1 beschrieben.

Ferner sind Vorrichtungen zur Freisetzung von Wirkstoffen auch für die Abwehr und/oder Bekämpfung von Insekten, wie Motten, einsetzbar. Die europäische Patentschrift EP 0 645 081 B1 beschreibt beispielsweise eine Vorrichtung zur insektiziden Behandlung und deren Verwendung in Wohnungen, vorzugsweise in Kleiderschränken, um die darin befindlichen Kleider vor einer Beschädigung durch Mottenbefall zu schützen.

Aufgabe der vorliegenden Erfindung war es nun, eine Vorrichtung zur Freisetzung von Wirkstoffen, insbesondere Duft- und Riechstoffen, bereitzustellen, welche zur Desodorierung und/oder Beduftung von Räumen, zur Abwehr und/oder Bekämpfung von Insekten als auch zur Behandlung von Erkrankungen der Atemwege sowie Kopfschmerzen und Schlafstörungen verwendet werden kann und über ein optimiertes Wirkstofffreisetzungsprofil verfügt. Ein solches optimiertes Wirkstofffreisetzungsprofil zeichnet sich insbesondere durch eine erhöhte Wirkstofffreisetzung aus.

Erfindungsgemäß wird diese Aufgabe gelöst durch eine Vorrichtung, umfassend einen Behälter mit Aufnahmekammer und darin befindlicher Wirkstoffzusammensetzung, eine den Behälter verschließende, die Diffusion der Wirkstoffe ermöglichende Membran und eine die Membran verschließende Schutzfolie, bei welcher die Membran aus einem Hüllmaterial umfassend eine Polyethylenfolie mit einem Gehalt von 5 bis 50 Gew.-% Ethylacrylat und/oder 5 bis 50 Gew.-% Ethylenvinylacetat, bezogen auf das Gesamtgewicht des Polyethylens, und die Schutzfolie aus einem Hüllmaterial umfassend eine Polyvinylalkoholfolie aufgebaut ist.

Überraschenderweise wurde gefunden, dass die Verwendung einer Membran aus einem Hüllmaterial umfassend eine Polyethylenfolie mit einem Gehalt von 5 bis 50 Gew.-% Ethylacrylat und/oder 5 bis 50 Gew.-% Ethylenvinylacetat verglichen mit herkömmlichen, im Stand der Technik verwendeten Membranen aus einem Hüllmaterial enthaltend eine Polyethylenfolie zu einer verbesserten Diffusion der in der Vorrichtung enthaltenen Wirkstoffe, insbesondere Duft- und Riechstoffe, führt. Neben der erhöhten Passierbarkeit für Wirkstoffe führt der erfindungsgemäße Zusatz von 5 bis 50 Gew.-% Ethylacrylat und/oder 5 bis 50 Gew.-% Ethylenvinylacetat zu der Polyethylenfolie jedoch auch zu einer erhöhten Instabilität der Membran, was dazu führt, dass die herkömmlichen, im Stand der Technik verwendeten Schutzfolien für Membranen aus Polyethylen nicht verwendet werden können, da diese die Membran beim Entfernen der Schutzfolie beschädigen. Es wurde jedoch ferner gefunden, dass sich eine Polyvinylalkoholfolie hervorragend als Schutzfolie für solche Membranen aus einem Hüllmaterial umfassend eine Polyethylenfolie mit einem Gehalt von 5 bis 50 Gew.-% Ethylacrylat und/oder 5 bis 50 Gew.-% Ethylenvinylacetat eignet. Eine solche Schutzfolie aus einem Hüllmaterial umfassend eine Polyvinylalkoholfolie ermöglicht eine perfekte Diffusionsbarriere für die Wirkstoffe und lässt sich leicht von der Membran entfernen, ohne diese zu beschädigen.

Ein erster Gegenstand der vorliegenden Anmeldung betrifft daher eine Vorrichtung zur Freisetzung wenigstens eines Wirkstoffs, umfassend
(i) einen Behälter (1) aus einem ersten Hüllmaterial, wobei der Behälter (1) eine Aufnahmekammer (2) zur Aufnahme einer den wenigstens einen Wirkstoff umfassenden Zusammensetzung bildet und an einer offenen Seite einen am Rand der Kammer umlaufenden Flansch (3) aufweist,
(ii) eine Membran (4) aus einem zweiten Hüllmaterial umfassend eine Polyethylenfolie mit einem Gehalt von 5 bis 50 Gew.-% Ethylacrylat und/oder 5 bis 50 Gew.-% Ethylenvinylacetat, bezogen auf das Gesamtgewicht des Polyethylens, wobei die Membran (4) an dem Flansch (3) des Behälters (1) fest angebracht ist, und
(iii) eine Schutzfolie (5) aus einem dritten Hüllmaterial umfassend eine Polyvinylalkoholfolie, wobei die Schutzfolie (5) auf der Membran (4) angebracht ist und mit der Hand ergriffen und durch Ziehen mit der Hand von der Membran (4) entfernt werden kann.

Erfindungsgemäß umfasst die Vorrichtung einen, vorzugsweise wannenartigen, bzw. topfartigen, Behälter (1) aus einem ersten Hüllmaterial der eine Aufnahmekammer (2) zur Aufnahme einer den wenigstens einen Wirkstoff umfassenden Zusammensetzung bildet und an einer offenen Seite einen am Rand der Kammer umlaufenden Flansch (3) aufweist. Unter dem Begriff "Flansch" ist hierbei ein Bereich an dem Behälter zu verstehen, an dem die Membran, welche letztendlich die offene Seite der Kammer verschließt, befestigt wird. In der Regel handelt es sich hierbei um einen an der dem Boden des Behälter gegenüberliegenden, offenen Seite der Aufnahmekammer umlaufenden flachen, üblicherweise nach außen gebogenen Rand. Unter einer festen Anbringung der Membran (4) an dem Flansch (3) des Behälters (1) ist hierbei zu verstehen, dass die Verbindung so fest ist, dass die Membran unter normalen Bedingungen, d. h. beispielsweise bei normalem Gebrauch, nicht mit der Hand ergriffen und/oder nicht durch Ziehen mit der Hand von dem Flansch (3) entfernt werden kann.

Das den Behälter (1) bildende erste Hüllmaterial ist für den wenigstens einen Wirkstoff undurchlässig. Vorzugsweise ist das den Behälter (1) bildende erste Hüllmaterial ausgewählt aus einer Polyacrylnitrilfolie, einer Polyesterfolie, einer Polypropylenfolie, einer Polyethylenfolie, und einer eine Polyacrylnitrilfolie, eine Polyesterfolie, eine Polypropylenfolie und/oder eine Polyethylenfolie umfassenden Verbundfolie.

Die Bezeichnung "Polyacrylnitrilfolie", wie hierin verwendet, bezeichnet Folien umfassend Polyacrylnitrile und Acrylnitril-Copolymere, wie beispielsweise Acrylnitril-Methylacrylat-Copolymere. Polyacrylnitrile bzw. Acrylnitril-Copolymere werden aus Acrylnitril bzw. Acrylnitril und einem weiteren Monomer nach unterschiedlichen im Stand der Technik bekannten. Polymerisationsverfahren (Substanz-, Lösungs-, Emulsions-, Suspensionspolymerisation) hergestellt und zu Folien bzw. Filmen verarbeitet. Gemäß einer bevorzugten Ausführungsform der vorliegenden Erfindung weist die Polyacrylnitrilfolie eine Dicke von 10 bis 600 µm, vorzugsweise 10 bis 400 µm, mehr bevorzugt 10 bis 300 µm und besonders bevorzugt 10 bis 150 µm auf. Besonders bevorzugt ist die Polyacrylnitrilfolie eine Acrylnitril-Methylacrylat-Copolymerfolie, wie die unter der Marke Barex™ von der Vistron Corp., Cleveland (Ohio) vertriebenen Folien.

Die Bezeichnung "Polypropylenfolie", wie hierin verwendet, bezeichnet Folien umfassend Polypropylene und Propylen-Copolymere. Verfahren zur Herstellung von Polypropylenen und Propylen-Copolymeren sind im Stand der Technik gut bekannt und umfassen beispielsweise die stereospezifische Polymerisation in der Gasphase oder in Suspension nach Natta. Die weitere Verarbeitung der Polypropylene und Propylen-Copolymere zu Folien und Filmen kann nach allen für Thermoplaste üblichen Verfahren erfolgen. Gemäß einer bevorzugten Ausführungsform der vorliegenden Erfindung weist die Polypropylenfolie eine Dicke von 10 bis 600 µm, vorzugsweise 10 bis 400 µm, mehr bevorzugt 10 bis 300 µm und besonders bevorzugt 10 bis 150 µm auf.

Im Rahmen der vorliegenden Erfindung bezeichnet die Bezeichnung "Polyethylenfolie", wie hierin verwendet, Folien umfassend Polyethylene und Ethylen-Copolymere. Polyethylene und Ethylen-Copolymere sind Polymere mit Gruppierungen des Typs - [CH₂-CH₂]- als charakteristische Grundeinheit der Polymerkette. Grundsätzlich sind im Stand der Technik zwei unterschiedlichen Methoden, das Hochdruck- und das Niederdruck-Verfahren, zur Herstellung der Polyethylene und Ethylen-Copolymere bekannt. Die aus diesen Verfahren resultierenden Produkte werden entsprechend als Hochdruck-Polyethylene/Hochdruck-Ethylen-Copolymere bzw. Niederdruck-Polyethylene/Niederdruck-Ethylen-Copolymere bezeichnet. Sie unterscheiden sich hauptsächlich hinsichtlich ihres Verzweigungsgrades und damit verbunden in ihrem Kristallinitätsgrad und ihrer Dichte. Beide Verfahren können als Lösungspolymerisation, Emulsionspolymerisation oder Gasphasenpolymerisation durchgeführt werden. Beim Hochdruck-Verfahren fallen Polyethylene und Ethylen-Copolymere mit niedriger Dichte (ca. 0,915 bis 0,935 g/cm³) und Kristallinitätsgraden von ca. 40 bis 50 % an, die man als LDPE-Typen (low density polyethylene) bezeichnet. Produkte mit höherer Molmasse und dadurch bedingter verbesserter Festigkeit und Streckbarkeit sind die HMW-LDPE-Typen (high molecular weight low density polyethylene). Durch Copolymerisation des Ethylens mit längerkettigen Olefinen, insbesondere mit Buten und Octen, kann der ausgeprägte Verzweigungsgrad der im Hochdruck-Verfahren hergestellten Polyethylene reduziert werden. Die Copolymere haben das Kurzzeichen LLD-PE (linear low density polyethylene). Die anschließende Verarbeitung der Polyethylene und Ethylen-Copolymere zu Folien und Filmen kann nach allen für Thermoplaste üblichen Verfahren erfolgen. Gemäß einer bevorzugten Ausführungsform der vorliegenden Erfindung weist die Polyethylenfolie eine Dicke von 10 bis 300 µm, vorzugsweise 10 bis 200 µm, mehr bevorzugt 10 bis 150 µm und besonders bevorzugt 10 bis 100 µm auf. Besonders bevorzugt ist die Polyethylenfolie eine Hochdruck-Polyethylenfolie, insbesondere eine Hochdruck-Polyethylenfolie vom LDPE-Typ, oder eine Polyethylenfolie mit 5 bis 50 Gew.-% Ethylacrylat und/oder 5 bis 50 Gew.-% Ethylenvinylacetat, bezogen auf das Gesamtgewicht des Polyethylens. Die Bezeichnung "Polyethylenfolie mit 5 bis 50 Gew.-% Ethylacrylat und/oder 5 bis 50 Gew.-% Ethylenvinylacetat, bezogen auf das Gesamtgewicht des Polyethylens", wie hierin verwendet, bezeichnet Polyethylenfolien mit 5 bis 50 Gew.-% Ethylacrylat und/oder 5 bis 50 Gew.-% Ethylenvinylacetat, vorzugsweise mit 5 bis 35 Gew.-% Ethylacrylat und/oder 5 bis 35 Gew.-% Ethylenvinylacetat oder mit 5 bis 30 Gew.-% Ethylacrylat und/oder 5 bis 30 Gew.-% Ethylenvinylacetat, mehr bevorzugt mit 5 bis 25 Gew.-% Ethylacrylat und/oder 5 bis 25 Gew.-% Ethylenvinylacetat oder mit 5 bis 20 Gew.-% Ethylacrylat und/oder 5 bis 20 Gew.-% Ethylenvinylacetat oder mit 10 bis 20 Gew.-% Ethylacrylat und/oder 10 bis 20 Gew.-% Ethylenvinylacetat, und besonders bevorzugt mit 10, 11, 12, 13, 14, 15, 16, 17, 18, 19 oder 20 Gew.-% Ethylacrylat und/oder 10, 11, 12, 13, 14, 15, 16, 17, 18, 19 oder 20 Gew.-% Ethylenvinylacetat, bezogen auf das Gesamtgewicht des Polyethylens.

Die Bezeichnung "eine Polyacrylnitrilfolie, eine Polypropylenfolie und/oder eine Polyethylenfolie umfassende Verbundfolie", wie hierin verwendet, bezeichnet Verbundfolien, die durch Blasen oder Laminieren wenigsten zweier Folien ausgewählt aus einer Polyacrylnitrilfolie, einer Polypropylenfolie und einer Polyethylenfolie hergestellt wurden. Verfahren zur Herstellung von Verbundfolien sind im Stand der Technik gut bekannt. Vorzugsweise ist die Verbundfolie eine aus einer Polyacrylnitrilfolie, einer Hochdruck-Polyethylenfolie und einer Polyethylenfolie mit 5 bis 50 Gew.-% Ethylacrylat und/oder 5 bis 50 Gew.-% Ethylenvinylacetat, bezogen auf das Gesamtgewicht des Polyethylens, hergestellte Verbundfolie oder eine aus einer Polypropylenfolie, einer Hochdruck-Polyethylenfolie und einer Polyethylenfolie mit 5 bis 50 Gew.-% Ethylacrylat und/oder 5 bis 50 Gew.-% Ethylenvinylacetat, bezogen auf das Gesamtgewicht des Polyethylens, hergestellte Verbundfolie.

Vorzugsweise weist die Wandung des Behälters (1) der erfindungsgemäßen Vorrichtung, d. h. das erste Hüllmaterial, eine Gesamtdicke von 10 bis 800 µm, vorzugsweise 10 bis 600 µm, mehr bevorzugt 10 bis 400 µm und besonders bevorzugt 10 bis 200 µm auf.

Der Behälter (1) der erfindungsgemäßen Vorrichtung kann eine beliebige Raumform eines regelmäßigen oder unregelmäßigen Körpers, Halbkörpers oder Teilkörpers aufweisen. Mögliche Raumformen umfassen Kugeln oder gestreckte Kugeln, Vierecke, Rechtecke, Tetraeder, Hexaeder, Oktaeder, Dodekaeder, lkosaeder sowie Halb- und Teilkörper davon. Denkbar sind weiterhin sternförmige Ausbildungen mit drei, vier, fünf, sechs oder mehr Spitzen oder völlig unregelmäßige Körper, die beispielsweise motivisch ausgestaltet sein können, sowie Halb- und Teilkörper davon. Als Motive eignen sich in Abhängigkeit von dem Einsatzgebiet der erfindungsgemässen Vorrichtung zum Beispiel Tierfiguren, wie Hunde, Pferde oder Vögel, florale Motive, wie Blumen, Blüten oder Bäume, oder die Darstellung von Früchten, wie Zitronen, Orangen oder Äpfel. Die motivische Ausgestaltung kann sich aber auch auf unbelebte Gegenstände, wie Fahrzeuge, Verkehrszeichen wie das Stoppzeichen, Werkzeuge, Haushaltsgegenstände oder Bekleidung beziehen. Besonders bevorzugt weist der Behälter (1) der erfindungsgemäßen Vorrichtung die Raumform einer Frucht oder eine Halb- oder Teilform davon auf.

Die Abmessungen und das Volumen des Behälters (1) und der darin befindlichen Aufnahmekammer (2) werden sich in erster Linie an dem späteren Verwendungszweck der erfindungsgemäßen Vorrichtung orientieren. In einer bevorzugten Ausführungsform der vorliegenden Anmeldung weist die Aufnahmekammer (2) ein Gesamtvolumen zwischen 0,1 und 1000 ml, vorzugsweise zwischen 0,2 und 100 ml, mehr bevorzugt zwischen 0,4 und 50 ml, besonders bevorzugt zwischen 0,6 und 30 ml und insbesondere zwischen 0,8 und 10 ml auf.

Die erfindungsgemäße Vorrichtung umfasst ferner eine Membran (4) aus einem zweiten Hüllmaterial umfassend eine Polyethylenfolie mit einem Gehalt von 5 bis 50 Gew.-% Ethylacrylat und/oder 5 bis 50 Gew.-% Ethylenvinylacetat, bezogen auf das Gesamtgewicht des Polyethylens, wobei die Membran (4) fest mit dem Flansch 3 des Behälters (1) verbunden ist. Das zweite Hüllmaterial ist für den wenigstens einen Wirkstoff durchlässig. Die Bezeichnung "Polyethylenfolie", wie hierin verwendet, bezeichnet, wie zuvor bereits beschrieben, Folien umfassend Polyethylene und Ethylen-Copolymere. Bevorzugte im Rahmen der vorliegenden Erfindung verwendete Polyethylenfolien umfassen Polyethylen- und Ethylen-Copolymerfolien vom LDPE-Typ, HMW-LDPE-Typ sowie LLD-PE-Typ.

Gemäß einer bevorzugten Ausführungsform der vorliegenden Erfindung ist das zweite Hüllmaterial ausgewählt ist aus einer Polyethylenfolie mit 5 bis 50 Gew.-% Ethylacrylat und/oder 5 bis 50 Gew.-% Ethylenvinylacetat, bezogen auf das Gesamtgewicht des Polyethylens, und einer eine Polyethylenfolie mit 5 bis 50 Gew.-% Ethylacrylat und/oder 5 bis 50 Gew.-% Ethylenvinylacetat, bezogen auf das Gesamtgewicht des Polyethylens, umfassenden Verbundfolie. Vorzugsweise weist die Polyethylenfolie mit 5 bis 50 Gew.-% Ethylacrylat und/oder 5 bis 50 Gew.-% Ethylenvinylacetat, bezogen auf das Gesamtgewicht des Polyethylens, oder die eine Polyethylenfolie mit 5 bis 50 Gew.-% Ethylacrylat und/oder 5 bis 50 Gew.-% Ethylenvinylacetat, bezogen auf das Gesamtgewicht des Polyethylens, umfassende Verbundfolie eine Dicke von 10 bis 300 µm, vorzugsweise 10 bis 200 µm, mehr bevorzugt 10 bis 150 µm und besonders bevorzugt 10 bis 100 µm auf.

Die Bezeichnung "eine Polyethylenfolie mit 5 bis 50 Gew.-% Ethylacrylat und/oder 5 bis 50 Gew.-% Ethylenvinylacetat, bezogen auf das Gesamtgewicht des Polyethylens, umfassende Verbundfolie", wie hierin verwendet, bezeichnet Verbundfolien, die durch Blasen oder Laminieren wenigstens zweier Folien, darunter eine Polyethylenfolie mit 5 bis 50 Gew.-% Ethylacrylat und/oder 5 bis 50 Gew.-% Ethylenvinylacetat, bezogen auf das Gesamtgewicht des Polyethylens, hergestellt wurden. Vorzugsweise ist die Verbundfolie eine aus einer Polyethylenfolie mit 5 bis 50 Gew.-% Ethylacrylat und/oder 5 bis 50 Gew.-% Ethylenvinylacetat, bezogen auf das Gesamtgewicht des Polyethylens, und einer Hochdruck-Polyethylenfolie hergestellte Verbundfolie.

Ferner sieht die vorliegende Erfindung vor, dass die Membran (4) eine Gesamtdicke von 10 bis 400 µm, vorzugsweise 10 bis 300 µm, mehr bevorzugt 10 bis 200 µm und besonders bevorzugt 10 bis 100 µm aufweist.

Die Membran (4) ist erfindungsgemäß zumindest so fest an dem Flansch (3) des Behälters (1) angebracht, dass sie bei normalen Bedingungen nicht mit der Hand ergriffen und/oder durch Ziehen mit der Hand von dem Flansch (3) entfernt werden kann. Vorzugsweise ist die Membran (4) durch Versiegeln fest an dem Flansch (3) des Behälters (1) angebracht. Geeignete Verfahren zur Versiegelung sind dem Fachmann bekannt und werden später näher beschrieben.

Des Weiteren umfasst die erfindungsgemäße Vorrichtung eine Schutzfolie (5) aus einem dritten Hüllmaterial umfassend eine Polyvinylalkoholfolie, wobei die Schutzfolie
(5) auf der Membran (4) angebracht ist und mit der Hand ergriffen und durch Ziehen mit der Hand von der Membran (4) entfernt werden kann. Das dritte Hüllmaterial ist für den wenigstens einen Wirkstoff undurchlässig. Die Bezeichnung "Polyvinylalkoholfolie", wie hierin verwendet, bezeichnet Folien umfassend Polyvinylalkohole. Charakterisiert werden Polyvinylalkohole von Seiten der Hersteller durch Angabe des Polymerisationsgrades, des Hydrolysegrades, der Verseifungszahl bzw. der Lösungsviskosität. Im Rahmen der vorliegenden Erfindung ist es bevorzugt, dass der Polyvinylalkohol der Polyvinylalkoholfolie einen Hydrolysegrad von 70 bis 100 Mol-%, vorzugsweise 80 bis 90 Mol-%, mehr bevorzugt 81 bis 89 Mol-% und besonders bevorzugt 82 bis 88 Mol-% aufweist. Ferner ist es bevorzugt, dass das Molekulargewicht des Polyvinylalkohols im Bereich von 10000 bis 100000 g/mol, vorzugsweise von 11000 bis 90000 g/mol, mehr bevorzugt von 12000 bis 80000 g/mol und besonders bevorzugt von 13000 bis 70000 g/mol liegt. Zudem liegt der Polymerisationsgrad des Polyvinylalkohols vorzugsweise zwischen 200 bis 2100, vorzugsweise zwischen 220 bis 1890, mehr bevorzugt zwischen 240 bis 1680 und besonders bevorzugt zwischen 260 bis 1500. Die vorstehend beschriebenen Polyvinylalkohole sind kommerziell erhältlich, beispielsweise unter dem Warenzeichen Mowiol® (Kuraray). Ein im Rahmen der vorliegenden Erfindung besonders geeigneter Polyvinylalkohol ist beispielsweise Mowiol® 8-88 und/oder 10-98.

Polyvinylalkohole sind abhängig vom Hydrolysegrad löslich in Wasser und wenigen stark polaren organischen Lösungsmitteln, wie Formamid, Dimethylformamid, Dimethylsulfoxid. Die Wasserlöslichkeit der Polyvinylalkohole lässt sich durch Nachbehandlung mit Aldehyden (Acetalisierung), durch Komplexierung mit Ni- oder Cu-Salzen oder durch Behandlung mit Dichromaten, Borsäure oder Borax verringern. Durch Wahl des Hydrolysegrades des Polyvinylalkohols der verwendeten Polyvinylalkoholfolie und gegebenenfalls durch eine Nachbehandlung ist es im Rahmen der vorliegenden Erfindung somit auch vorgesehen, dass die Schutzfolie (5) durch Abwaschen mit Wasser, beispielsweise bei Gebrauch der Vorrichtung in einer Geschirrspülmaschine oder Waschmaschine oder einem WC, entfernbar ist.

Vorzugsweise ist das dritte Hüllmaterial umfassend eine Polyvinylalkoholfolie ausgewählt aus einer Polyvinylalkoholfolie und einer eine Polyvinylalkoholfolie umfassenden Verbundfolie. Ferner ist es gemäß vorliegender Erfindung bevorzugt, dass die Polyvinylalkoholfolie eine Dicke von 10 bis 500 µm, vorzugsweise 10 bis 400 µm, mehr bevorzugt 10 bis 200 µm und besonders bevorzugt 10 bis 150 µm aufweist.

Zudem sieht die vorliegende Erfindung vor, dass die Schutzfolie (5) eine Gesamtdicke von 10 bis 500 µm, vorzugsweise 10 bis 400 µm, mehr bevorzugt 10 bis 200 µm und besonders bevorzugt 10 bis 150 µm aufweist.

Die Schutzfolie (5) ist erfindungsgemäß so auf der Membran (4) angebracht, dass sie mit der Hand ergriffen und durch Ziehen mit der Hand von der Membran (4) entfernt werden kann. Vorzugsweise ist die Schutzfolie (5) durch Versiegeln ganzflächig fest auf der Membran (4) angebracht. Besonders bevorzugt ist die Schutzfolie (5) in dem dem Bereich des Flansches (3) des Behälters (1) entsprechenden Bereich und/oder in dem innerhalb des Bereichs des Flansches (3) des Behälters (1) liegenden Bereich auf der Membran (4) angebracht. Geeignete Verfahren zur Versiegelung sind dem Fachmann bekannt und werden später näher beschrieben. Wichtig bei der Versiegelung ist jedoch, dass die durch die Versiegelung erzielte Haftung der Membran (4) auf dem Behälter (1) bei weitem stärker ist, als die durch die Versiegelung erzielte Haftung der Schutzfolie (5) auf der Membran (4), so dass die Schutzfolie (5) durch Ziehen mit der Hand entfernt werden kann, ohne dabei die Membran (4) von dem Behälter (1) abzuziehen oder zu beschädigen.

In einer besonders bevorzugten Ausführungsform der vorliegenden Erfindung umfasst die Vorrichtung einen Behälter (1) aus einem ersten Hüllmaterial ausgewählt aus einer eine Barex™-Folie, eine Hochdruck-Polyethylenfolie und eine Polyethylenfolie mit 5 bis 50 Gew.-% Ethylacrylat und/oder 5 bis 50 Gew.-% Ethylenvinylacetat, bezogen auf das Gesamtgewicht des Polyethylens, umfassenden Verbundfolie und einer eine Polypropylenfolie, eine Hochdruck-Polyethylenfolie und eine Polyethylenfolie mit 5 bis 50 Gew.-% Ethylacrylat und/oder 5 bis 50 Gew.-% Ethylenvinylacetat, bezogen auf das Gesamtgewicht des Polyethylens, umfassenden Verbundfolie, eine Membran (4) aus einem zweiten Hüllmaterial ausgewählt aus einer eine Polyethylenfolie mit 5 bis 50 Gew.-% Ethylacrylat und/oder 5 bis 50 Gew.-% Ethylenvinylacetat, bezogen auf das Gesamtgewicht des Polyethylens, und eine Hochdruck-Polyethylenfolie umfassenden Verbundfolie, und eine Schutzfolie (5) aus einem dritten Hüllmaterial ausgewählt aus einer Polyvinylalkoholfolie.

Zudem sieht die vorliegende Erfindung ferner vor, dass die erfindungsgemäße Vorrichtung gegebenenfalls eine Aufhängeeinrichtung aufweist. Diese Aufhängevorrichtung ermöglicht das einfache Anbringen der Vorrichtung, beispielsweise in Schränken, Kraftfahrzeuginnenräumen, Geschirrspülmaschinen, Kühlschränken, etc.

Gemäß einer bevorzugten Ausführungsform der vorliegenden Erfindung enthält die Vorrichtung zudem eine in der Aufnahmekammer (2) befindliche Zusammensetzung umfassend den wenigstens einen Wirkstoff. Vorzugsweise ist der wenigstens eine Wirkstoff ausgewählt aus Duft- und Riechstoffen sowie beliebigen Kombinationen davon. Die Bezeichnung "Duft- und Riechstoffe", wie hierin verwendet, umfasst einzelne Duft- und Riechstoffverbindungen, wie beispielsweise synthetische Produkte vom Typ der Ester, Ether, Aldehyde, Ketone, Alkohole und Kohlenwasserstoffe als auch natürliche Duft- und Riechstoffe bzw. Duft- und Riechstoffgemische. Beispiele für Duft- und Riechstoffverbindungen vom Ester-Typ sind unter anderem Benzylacetat, Phenoxyethylisobutyrat, p-tert-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Duft- und Riechstoffverbindungen vom Ether-Typ zählen beispielsweise Benzylethylether, zu den Duft- und Riechstoffverbindungen vom Aldehyd-Typ lineare Alkanale mit 8 bis 18 Kohlenstoffatomen, Citral, Citronellal, Citronellyloxyacetaldehyd, Cyclamenaldehyd, Hydroxycitronellal, Lilial und Bourgeonal. Des Weiteren sind Beispiele von Duft- und Riechstoffverbindungen vom Keton-Typ die Jonone, α-Isomethylionon und Methylcedrylketon, vom Alkohol-Typ Anethol, Citronellol, Eugenol, Geraniol, Linalool, Phenylethylalkohol und Terpineol und vom Kohlenwasserstoff-Typ Terpene, wie Limonen und Pinen. Beispiele für natürliche Duft- und Riechstoffe bzw. Duft- und Riechstoffgemische, die aus pflanzlichen Quellen zugänglich sind oder synthetisch hergestellt werden, sind Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl, Labdanumöl, Orangenblütenöl, Neroliol, Orangenschalenöl, Sandelholzöl und Lavendel-Ceder und deren Inhaltsstoffe.

Beispiele für haftfeste Duft- und Riechstoffverbindungen, die im Rahmen der vorliegenden Erfindung verwendet werden können, umfassen insbesondere ätherische Öle, wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Campheröl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Argumenöl, Orangenöl, Origanumöl, Palmarosaöl, Patschuliöl, Perubalsamöl, Petitgrainöl, Pfefferöl, Pfefferminzöl, Pimentöl, Pineöl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronenöl, Cedernöl sowie Zypressenöl. Beispiele für höher siedende bzw. feste Duft- und Riechstoffverbindungen umfassen insbesondere Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Isoborneol, Isobornylacetat, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinondimethylether, Hydroxyzimtaldehyd, Hydroxyzimtalkohol, Indol, Iron, Isoeugenol, Isoeugenolmethylether, Isosafrol, Jasmon, Campher, Carvakrol, Carvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Musketon, β-Naphtholethylether, Nerol, Nitrobenzol, n-Nonylaldehyd, Nonylakohol, n-Octylaldehyd, p-Oxy-Acetophenon, Pentadekanolid, β-Phenylethylalkohol, Phenylacetaldehyddimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimtalkohol, Zimtsäure, Zimtsäureethylester, Zimtsäurebenzylester. Zu den leichter flüchtigen Duft- und Riechstoffverbindungen zählen insbesondere Alkyisothiocyanate (Alkylsenföle), Butandion, Limonen, Linalool, Linaylacetat und -propionat, Menthol, Menthon; Methyl-n-heptenon, Phellandren, Phenylacetaldehyd, Terpinylacetat, Citral und Citronellal. Bevorzugt werden jedoch Mischungen verschiedener der oben genannten Duft- und Riechstoffverbindungen verwendet, die gemeinsam eine ansprechende Duftnote erzeugen.

Ferner sieht die vorliegende Erfindung vor, dass die in der Aufnahmekammer (2) befindliche Zusammensetzung zusätzlich wenigstens eine weitere Verbindung ausgewählt aus Lösungsmitteln, Verdampfungsmitteln und Farbstoffen und Konservierungsmitteln enthält. Geeignete Lösungsmittel zur Verwendung in Verbindung mit Zusammensetzungen umfassend wenigstens einen der oben genannten Wirkstoffe sind dem Fachmann gut bekannt und umfassen beispielsweise organische Lösungsmittel, wie Alkane (Pentan, Hexan), Ethylmethylketon, Ethylacetat, Alkohole und isoparaffinische Kohlenwasserstoffe.

Bei dem Verdampfungsmittel, das gemäß vorliegender Erfindung verwendet werden kann, handelt es sich um herkömmliche im Stand der Technik bekannte Verdampfungsmittel. Vorzugsweise handelt es sich dabei um ein Verdampfungsmittel, dessen Verdampfungsgeschwindigkeit mindestens zwei mal, vorzugsweise mindestens drei mal so groß ist wie die Verdampfungsgeschwindigkeit des wenigstens einen Wirkstoffs. Besonders bevorzugt ist das Verdampfungsmittel ausgewählt aus der Gruppe bestehend aus Hexylacetat, Isobutylacetat, Cyclohexylacetat, Phenylacetat, Isononylacetat, Linalylacetat, Benzylacetat, Ethylbutyrat, Campher, Ethylcaproat, Methylcaproat, Limonen, Allylheptanoat, Methylhexanoat, Ethylisobutyrat, Pinen, Kohlenwasserstoffe und Cyclohexen.

Geeignete Farbstoffe, die im Rahmen der vorliegenden Erfindung verwendet werden können, umfassen sämtliche Farbstoffe, die dem Fachmann als geeignet zum Einfärben von Zusammensetzungen umfassend wenigstens einen der oben genannten Wirkstoffe bekannt sind. Es ist bevorzugt, den Farbstoff entsprechend des verwendeten Wirkstoffs auszuwählen. Beispielsweise weisen Partikel mit Zitronenduft vorzugsweise eine gelbe Farbe auf, während für Partikel mit Apfel- oder Kräuterduft eine grüne Farbe bevorzugt wird. Bevorzugte Farbstoffe besitzen eine hohe Lagerstabilität und Unempfindlichkeit gegenüber den übrigen Inhaltsstoffen der Zusammensetzung sowie gegen Licht. Wird die erfindungsgemäße Vorrichtung im Zusammenhang mit der Textil- oder Geschirrreinigung eingesetzt, sollten die eingesetzten Farbstoffe keine ausgeprägte Substantivität gegenüber Textilfaser, Glas, Kunststoffgeschirr oder Keramik aufweisen, um diese nicht anzufärben. Geeignete Farbstoffe und Farbstoffgemische sind unter verschiedenen Handelsnamen kommerziell erhältlich und werden unter anderem von den Firmen BASF AG, Ludwigshafen, Bayer AG, Leverkusen, Clariant GmbH, DyStar Textilfarben GmbH & Co. Deutschland KG, Les Colorants Wackherr SA und Ciba Specialty Chemicals angeboten. Zu den geeigneten fettlöslichen Farbstoffen und Farbstoffgemischen zählen beispielsweise Solvent Blue 35, Solvent Green 7, Solvent Orange 1, Sandoplast Blau 2B, Fettgelb 3G, Iragon® Red SRE 122, Iragon® Green SGR 3, Solvent Yellow 33 und Solvent Yellow 16, es können aber auch andere Farbstoffe verwendet werden.

Geeignete Konservierungsmittel, die in Verbindung mit Zusammensetzungen umfassend wenigstens einen der oben genannten Wirkstoffe verwendet werden können, sind dem Fachmann gut bekannt und umfassen beispielsweise Antioxidationsmittel, Desinfektionsmittel und Biozidstoffe, wie Biozidstoffe, die durch die Membran diffundieren (z.B. Transfluthrin).

Ein zweiter Gegenstand der vorliegenden Anmeldung betrifft ein Verfahren zur Herstellung einer erfindungsgemäßen Vorrichtung, umfassend
(a) das Erzeugen des Behälters (1) durch Tiefziehen des ersten Hüllmaterials unter Ausbildung der Aufnahmekammer (2),
(b) das Erzeugen der Membran (4) durch Versiegeln der Aufnahmekammer (2) mit dem zweiten Hüllmaterial, wobei das Versiegeln im Bereich des Flansches (3) des Behälters (1) erfolgt und wobei eine kleine Stelle in diesem Bereich zum Befüllen der Aufnahmekammer (2) mit einer Zusammensetzung umfassend den wenigstens einen Wirkstoff offen bleibt,
(c) das Befüllen der Aufnahmekammer (2) mit der Zusammensetzung umfassend den wenigstes einen Wirkstoff, und
(d) das Erzeugen der Schutzfolie (5) durch Versiegeln oder Laminieren der Membran (4) mit dem dritten Hüllmaterial, wobei das Versiegeln in dem dem Bereich des Flansches (3) des Behälters (1) entsprechenden Bereich der Membran (4) und/oder in dem innerhalb dieses Bereiches liegenden Bereich der Membran (4) erfolgt.

Erfindungsgemäß umfasst das Verfahren in Schritt (a) das Erzeugen des Behälters (1) durch Tiefziehen des ersten Hüllmaterials unter Ausbildung der Aufnahmekammer (2). Mit der Bezeichnung "Tiefziehen" oder "Tiefziehverfahren", wie hierin verwendet, werden im Rahmen der vorliegenden Anmeldung Verfahren zur Verarbeitung von Hüllmaterialien bezeichnet, bei welchen diese nach optionaler Vorbehandlung durch Wärme und/oder Lösungsmittel mittels einer entsprechend geformten Matrize in Form gebracht werden. Dabei kann das Hüllmaterial beispielsweise als Platte, Folie oder Film zwischen die beiden Teile des Werkzeugs, das Positiv und das Negativ, eingebracht und durch Zusammendrücken dieser Teile verformt werden. Die Verformung kann jedoch auch ohne Einsatz eines Negativ-Werkzeugs durch Einwirkung eines Vakuums und/oder von Druckluft erfolgen. Aus der Reihe der beschriebenen Tiefziehverfahren werden solche Verfahren bevorzugt, bei denen das Hüllmaterial in Form einer Folie über einer mit Vertiefungen versehenen Matrize bereitgestellt und durch Einwirkung von Druckluft von der Oberseite der Folien oder durch Wirkung eines Vakuums von der Unterseite der Folien, besondere bevorzugt unter gleichzeitiger Einwirkung von Druckluft und Vakuum in die Vertiefungen der Matrize eingebracht und entsprechend der Form der Vertiefung ausgeformt wird. Besonders vorteilhafte Verfahren zeichnen sich dabei dadurch aus, dass die Folie vor dem Verformen durch Einwirken von Wärme und/oder Lösungsmitteln vorbehandelt wird. In einer weiteren bevorzugten Verfahrensvariante wird eine Folie nach optionaler Vorbehandlung (Lösungsmittel, Wärme) durch Einwirkung eines Stempels und/oder durch die Einwirkung der Gewichtskraft des Füllguts formgebend in die Vertiefung einer Matrize gepresst. Die Einwirkung von Wärme und/oder Lösungsmitteln auf das Hüllmaterial dient dessen erleichterter plastischen Verformung. Die Erwärmung des Hüllmaterials kann dabei beispielsweise durch Wärmestrahlung, Heißluft oder besonders bevorzugt durch direkten Kontakt mit einer Heizplatte erfolgen. Alternativ können zur Erwärmung des Hüllmaterials aber auch beheizte Rollen oder Walzen eingesetzt werden. Die Dauer der Wärmebehandlung sowie die Temperatur der eingesetzten Wärmestrahlung, Heißluft oder Heizplattenoberfläche ist dabei naturgemäß von der Art des eingesetzten Hüllmaterials abhängig. Der Flansch des Behälters wird bei einem solchen Herstellungsprozess vorzugsweise erzeugt, in dem beim Ausstanzen oder Ausschneiden der tiefgezogene Kammer aus der aus dem ersten Hüllmaterial bestehenden Platte oder Folie einfach darauf geachtet wird, dass ein flacher umlaufender Rand des ersten Hüllmaterials um den tiefgezogenen Bereich stehen bleibt.

Ferner umfasst das erfindungsgemäße Verfahren in Schritt (b) das Erzeugen der Membran (4) durch Versiegeln oder Laminieren der Aufnahmekammer (2) mit dem zweiten Hüllmaterial, wobei das Versiegeln im Bereich des Flansches (3) des Behälters (1) erfolgt und wobei eine kleine Stelle in diesem Bereich zum Befüllen der Aufnahmekammer (2) mit einer Zusammensetzung umfassend den wenigstens einen Wirkstoff offen bleibt, und in Schritt (c) das Befüllen der Aufnahmekammer (2) mit der Zusammensetzung umfassend den wenigstes einen Wirkstoff. Die Versiegelung erfolgt dabei vorzugsweise durch Einwirkung von Druck und/oder Wärme und/oder Lösungsmitteln. Geeignete Siegeltemperaturen sind beispielsweise 80 bis 200 °C, vorzugsweise Temperaturen im Bereich von 120 bis 170 °C, insbesondere im Bereich von 130 bis 150 °C. Als Siegeldruck haben sich Drucke im Bereich von 250 bis 800 kPa, vorzugsweise 272 bis 554 kPa, besonders bevorzugt von 341 bis 481 kPa als vorteilhaft erwiesen. Die Siegelzeiten betragen vorzugsweise mindestens 0,3 Sekunden, vorzugsweise zwischen 0,4 und 4 Sekunden. Siegeltemperaturen, -drucke und Siegelzeiten werden neben dem eingesetzten Hüllmaterial auch durch die eingesetzte Siegelmaschine bestimmt. Die Siegelnähte weisen vorzugsweise eine Breite zwischen 0,5 und 7,0 cm, mehr bevorzugt zwischen 1,0 und 2,0 cm und besonders bevorzugt zwischen 1,5 und 15 mm auf. Als ausreichend haltbar haben sich insbesondere Siegelnähte mit einer Breite oberhalb 1 cm, vorzugsweise oberhalb 0,5 cm, mehr bevorzugt oberhalb 1 cm und besonders bevorzugt oberhalb 5 mm erwiesen.

Des Weiteren umfasst das erfindungsgemäße Verfahren in Schritt (d) das Erzeugen der Schutzfolie (5) durch Versiegeln der Membran (4) mit dem dritten Hüllmaterial, wobei das Versiegeln in dem dem Bereich des Flansches (3) des Behälters (1) entsprechenden Bereich der Membran (4) und/oder in dem innerhalb dieses Bereiches liegenden Bereich der Membran (4) erfolgt. Dabei erfolgt vorteilhafterweise gleichzeitig auch das Verschließen der im Schritt (d) belassenen Befüllöffnung. Wie bereits zuvor beschrieben, erfolgt die Versiegelung vorzugsweise durch Einwirkung von Druck und/oder Wärme und/oder Lösungsmitteln. Geeignete Siegeltemperaturen, Siegeldrucke und Siegelzeiten entsprechen den oben Genannten. Vorzugsweise erfolgt die Versiegelung in dem dem Bereich des Flansches (3) des Behälters (1) entsprechenden Bereich der Membran (4) und in dem innerhalb dieses Bereichs liegenden Bereich der Membran (4). Besonders bevorzugt erfolgt die Versiegelung dabei so, dass ein Teil der Schutzfolie (5) nach der Versiegelung mit der Hand ergriffen und durch Ziehen mit der Hand von der Membran (4) entfernt werden kann.

Gemäß einer weiteren bevorzugten Ausführungsform der vorliegenden Erfindung kann der bei dem oben erläuterten Verfahren mit (c) bezeichnete Schritt vor dem mit (b) bezeichneten Schritt durchgeführt werden. Dabei wird die Aufnahmekammer (2) zunächst mit der Zusammensetzung umfassend den wenigstens einen Wirkstoff befüllt und nachfolgend durch Versiegeln mit dem zweiten Hüllmaterial verschlossen, wobei die Versiegelung so erfolgt, dass der Behälter (1) im Bereich des Flansches (3) vollständig verschlossen wird.

Ein weiterer Gegenstand der vorliegenden Anmeldung betrifft die Verwendung der erfindungsgemäßen Vorrichtung zur Freisetzung wenigstens einen Wirkstoffs, wobei die Verwendungsmöglichkeiten sehr zahlreich und umfassend sind. So kann die erfindungsgemäße Vorrichtung beispielsweise zur Desodorierung und/oder Beduftung von Räumen verwendet werden. Insbesondere eignet sich die erfindungsgemäße Vorrichtung zur Desodorierung und/oder Beduftung von Gebäudeinnenräumen, wie Büroräumen, Wohnräumen, Küchen, Toiletten, etc., von Fahrzeuginnenräumen, wie Kraftfahrzeuginnenräumen, Flugzeuginnenräumen, Zuginnenräumen, etc., Geschirrspülmaschineninnenräumen, Waschmaschineninnenräumen, Kühlschrankinnenräumen, Mülleimerinnenräumen, Innenräumen von Tiertoiletten, wie Katzentoiletten, etc, und Innenräumen von Einrichtungsgegenständen, wie Schrankinnenräumen, Kommodeninnenräumen, etc.

Ferner kann die erfindungsgemäße Vorrichtung zur Abwehr und/oder Bekämpfung von Insekten, wie Stechmücken oder Motten, verwendet werden. Vorzugsweise findet die erfindungsgemäße Vorrichtung zur Abwehr und/oder Bekämpfung von Motten in Kleiderschränken oder Vorratsschränken Anwendung.

Zudem kann die erfindungsgemäße Vorrichtung jedoch auch zur Behandlung von Erkrankungen des Atemwegssystems oder von Kopfschmerzen und Schlafstörungen eingesetzt werden. Dabei wird die erfindungsgemäße Vorrichtung vorzugsweise in der Nähe eines zu behandelnden Patienten, wie beispielsweise auf einem Tisch oder Nachttisch, aufgestellt, so dass der von der Vorrichtung freigesetzte wenigstens eine Wirkstoff durch das Atmungssystem der Patienten aufgenommen werden kann.

Weiterhin soll die Erfindung durch die nachfolgenden Figuren und Beispiele näher veranschaulicht werden.

### Figuren

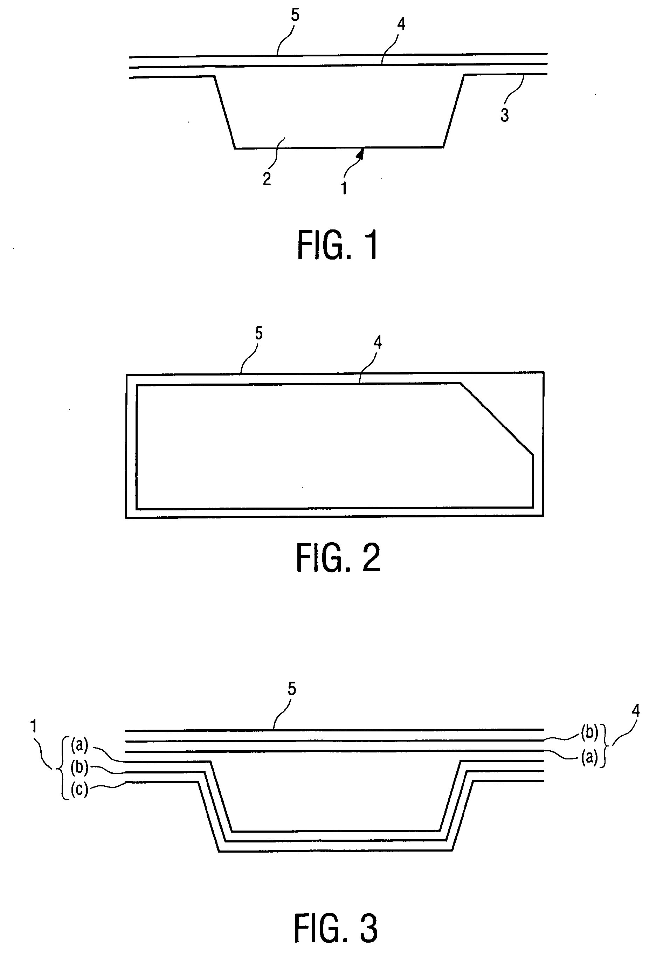

**Fig. 1** zeigt eine schematische Abbildung (Seitenansicht) einer erfindungsgemäßen Vorrichtung umfassend einen Behälter (1) mit einem von einem flachem, nach außen gebogenem Rand gebildeten Flansch (3), einer Aufnahmekammer (2), einer Membran (4) und einer Schutzfolie (5).
**Fig. 2** zeigt eine schematische Abbildung (Draufsicht) einer erfindungsgemäßen Vorrichtung mit einer an dem Flansch (3) des Behälters (1) fest angebrachten (beispielsweise mittels Versiegelung) Membran (4) und einer auf der Membran (4) fest angebrachten (beispielsweise mittels Versiegelung) Schutzfolie (5), wobei die Schutzfolie (5) auf der Membran (4) so angebracht ist, dass sie an einem Ende (Abzugsecke) mit der Hand ergriffen und durch Ziehen mit der Hand von der Membran (4) entfernt werden kann.
**Fig. 3** zeigt eine schematische Abbildung (Seitenansicht) einer bevorzugten erfindungsgemäßen Vorrichtung. Bei dieser Vorrichtung ist der Behälter (1) aus einer Verbundfolie, hergestellt aus (a) einer Polyethylenfolie mit 5 bis 50 Gew.-% Ethylacrylat und/oder 5 bis 50 Gew.-% Ethylenvinylacetat, bezogen auf das Gesamtgewicht des Polyethylens, (b) einer Hochdruck-Polyethylenfolie und (c) einer Barex™-Folie oder Polypropylenfolie, aufgebaut. Die Membran (4) besteht aus einer Verbundfolie, hergestellt aus (a) einer Polyethylenfolie mit 5 bis 50 Gew.-% Ethylacrylat und/oder 5 bis 50 Gew.-% Ethylenvinylacetat, bezogen auf das Gesamtgewicht des Polyethylens, und (b) einer Hochdruck-Polyethylenfolie. Die Schutzfolie (5) ist eine Polyvinylalkoholfolie.

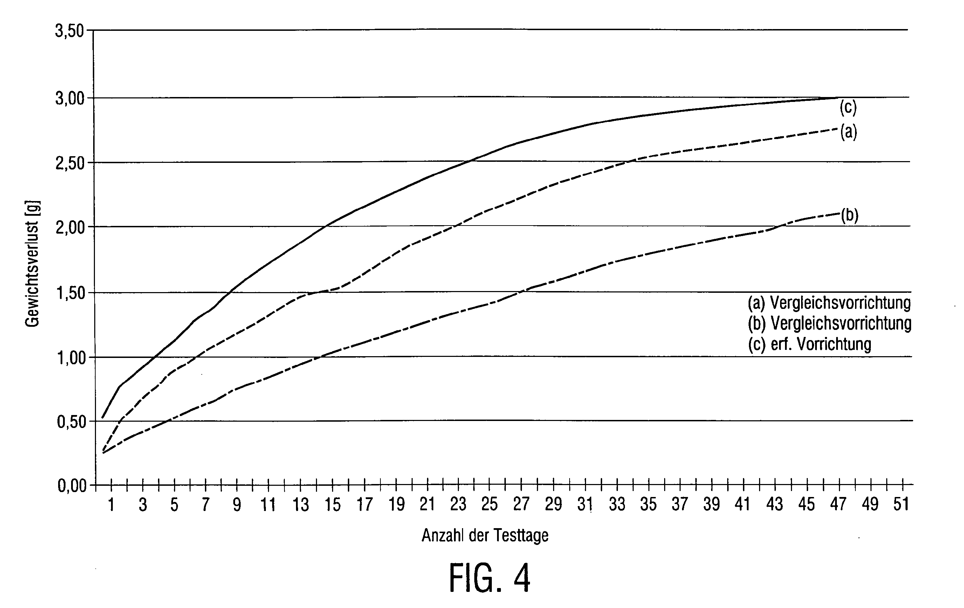

**Fig. 4** zeigt einen Vergleich des Wirkstofffreisetzungsprofils der erfindungsgemäßen Vorrichtung verglichen mit zwei Vorrichtungen aus dem Stand der Technik.

### Beispiele

### Beispiel 1: Herstellung einer erfindungsgemäßen Vorrichtung

Mit einer Verbundfolie, die durch Blasen oder Laminieren aus einer Barex™-Folie (Dicke: 50 bis 450 µm), einer Hochdruck-Polyethylenfolie (Dicke: 10 bis 150 µm) und einer Polyethylenfolie mit 6 Gew.-% Ethylacrylat, bezogen auf das Gesamtgewicht des Polyethylens, (Dicke: 40 µm) hergestellt wurde, wurde ein Behälter durch ein herkömmliches, im Stand der Technik bekanntes Tiefziehverfahren hergestellt.

Auf den Rand des Behälter wurde eine Membran bestehend aus einer Verbundfolie, die durch Blasen oder Laminieren aus einer Hochdruck-Polyethylenfolie (Dicke: 10 bis 50 µm) und einer Polyethylenfolie mit 10 bis 35 Gew.-% Ethylacrylat und/oder 10 bis 35 Gew.-% Ethylenvinylacetat, bezogen auf das Gesamtgewicht des Polyethylens, (Dicke: 40 µm) hergestellt wurde, so aufgesiegelt, dass in einem kleinen Bereich zwischen Behälter und Membran ein Füllloch offen blieb. Der Behälter wurde anschließend mit einer der in Beispiel 6 beschriebenen Wirkstoffzusammensetzungen befüllt.

Nun wurde eine Schutzfolie bestehend aus einer Polyvinylalkoholfolie (Dicke: 10 bis 500 µm) vollflächig oder randflächig und/oder unter Berücksichtigung einer Abzugsecke heiß auf die Membran aufgesiegelt, so dass auch das Füllloch verschlossen wurde.

### Beispiel 2: Herstellung einer erfindungsgemäßen Vorrichtung

Mit einer Verbundfolie, die durch Blasen oder Laminieren aus einer Polypropylen-Folie (Dicke: 50 bis 450 µm), einer Hochdruck-Polyethylenfolie (Dicke: 10 bis 150 µm) und einer Polyethylenfolie mit 6 Gew.-% Ethylacrylat, bezogen auf das Gesamtgewicht des Polyethylens, (Dicke: 40 µm) hergestellt wurde, wurde ein Behälter durch ein herkömmliches, im Stand der Technik bekanntes Tiefziehverfahren hergestellt.

Auf den Rand des Behälter wurde eine Membran bestehend aus einer Verbundfolie, die durch Blasen oder Laminieren aus einer Hochdruck-Polyethylenfolie (Dicke: 10 bis 50 µm) und einer Polyethylenfolie mit 10 bis 35 Gew.-% Ethylacrylat und/oder 10 bis 35 Gew.-% Ethylenvinylacetat, bezogen auf das Gesamtgewicht des Polyethylens, (Dicke: 40 µm) hergestellt wurde, so aufgesiegelt, dass in einem kleinen Bereich zwischen Behälter und Membran ein Füllloch offen blieb. Der Behälter wurde anschließend mit einer der in Beispiel 6 beschriebenen Wirkstoffzusammensetzungen befüllt.

Nun wurde eine Schutzfolie bestehend aus einer Polyvinylalkoholfolie (Dicke: 10 bis 500 µm) vollflächig oder randflächig und/oder unter Berücksichtigung einer Abzugsecke heiß auf die Membran aufgesiegelt, so dass auch das Füllloch verschlossen wurde.

### Beispiel 3: Herstellung einer erfindungsgemäßen Vorrichtung

Mit einer Polypropylenfolie (Dicke: 300 µm) wurde ein Behälter durch ein herkömmliches, im Stand der Technik bekanntes Tiefziehverfahren hergestellt.

Auf den Rand des Behälter wurde eine Membran bestehend aus einer Verbundfolie, die durch Blasen oder Laminieren aus einer Hochdruck-Polyethylenfolie (Dicke: 10 bis 50 µm) und einer Polyethylenfolie mit 10 bis 35 Gew.-% Ethylacrylat und/oder 10 bis 35 Gew.-% Ethylenvinylacetat, bezogen auf das Gesamtgewicht des Polyethylens, (Dicke: 40 µm) hergestellt wurde, so aufgesiegelt, dass in einem kleinen Bereich zwischen Behälter und Membran ein Füllloch offen blieb. Der Behälter wurde anschließend mit einer der in Beispiel 6 beschriebenen Wirkstoffzusammensetzungen befüllt.

Nun wurde eine Schutzfolie bestehend aus einer Polyvinylalkoholfolie (Dicke: 10 bis 500 µm) vollflächig oder randflächig und/oder unter Berücksichtigung einer Abzugsecke heiß auf die Membran aufgesiegelt, so dass auch das Füllloch verschlossen wurde.

### Beispiel 4: Herstellung einer erfindungsgemäßen Vorrichtung

Mit einer Polyamidfolie (Dicke: 30 µm) wurde ein Behälter durch ein herkömmliches, im Stand der Technik bekanntes Tiefziehverfahren hergestellt.

Auf den Rand des Behälter wurde eine Membran bestehend aus einer Verbundfolie, die durch Blasen oder Laminieren aus einer Hochdruck-Polyethylenfolie (Dicke: 10 bis 50 µm) und einer Polyethylenfolie mit 10 bis 35 Gew.-% Ethylacrylat und/oder 10 bis 35 Gew.-% Ethylenvinylacetat, bezogen auf das Gesamtgewicht des Polyethylens, (Dicke: 40 µm) hergestellt wurde, so aufgesiegelt, dass in einem kleinen Bereich zwischen Behälter und Membran ein Füllloch offen blieb. Der Behälter wurde anschließend mit einer der in Beispiel 6 beschriebenen Wirkstoffzusammensetzungen befüllt.

Nun wurde eine Schutzfolie bestehend aus einer Polyvinylalkoholfolie (Dicke: 10 bis 500 µm) vollflächig oder randflächig und/oder unter Berücksichtigung einer Abzugsecke heiß auf die Membran aufgesiegelt, so dass auch das Füllloch verschlossen wurde.

### Beispiel 5: Wirkstofffreisetzungsprofil der erfindungsgemäßen Vorrichtung

Zur Untersuchung des Wirkstofffreisetzungsprofils der erfindungsgemäßen Vorrichtung wurden folgende Vorrichtungen wie unten beschrieben hergestellt und mit einer Wirkstoffzusammensetzung, umfassend Lavandinöl bzw. Zedernholzöl, zur Abwehr und/oder Bekämpfung von Motten befüllt.

### a) Vergleichsvorrichtung aus dem Stand der Technik:

Mit einer Verbundfolie, hergestellt aus einer Barex™-Folie (Dicke: 250 µm) und einer Hochdruck-Polyethylenfolie (Dicke: 50 µm), wurde ein Behälter gezogen. Auf den Behälter wurde eine Membran aus einer Verbundfolie, hergestellt aus einer Polyethylenfolie mit 15 Gew.-% Ethylacrylat (Dicke: 130 µm) und einer Hochdruck-Polyethylenfolie (Dicke: 20 µm), mit einer Außenseite aus Papier 32 g/m² (zum Schutz der Membran) heiß so aufgesiegelt, dass zwischen Behälter und Membran ein Füllloch offen blieb. Nach dem Befüllen des Behälters mit der Wirkstoffzusammensetzung wurde eine Schutzfolie, bestehend aus einer Polyethylenfolie (Dicke: 19 µm), lackkaschiert mit einer Polyethylenfolie (Dicke: 80 µm) und verleimt mit lösungsmittelbeständigem Kleber, auf die Papierseite der Membran heiß aufgesiegelt, so dass auch das Füllloch verschlossen wurde und eine Abzugsecke zum Entfernen der Schutzfolie entstand.

### b) Vergleichsvorrichtung aus dem Stand der Technik:

Mit einer Verbundfolie, hergestellt aus einer Barex™-Folie (Dicke: 250 µm) und einer Hochdruck-Polyethylenfolie (Dicke: 50 µm), wurde ein Behälter gezogen. Auf den Behälter wurde eine Membran aus einer Verbundfolie, hergestellt aus einer Polyethylenfolie (Dicke: 130 µm) und einer Hochdruck-Polyethylenfolie (Dicke: 20 µm), heiß so aufgesiegelt, dass zwischen Behälter und Membran ein Füllloch offen blieb. Nach dem Befüllen des Behälters mit der Wirkstoffzusammensetzung wurde eine Schutzfolie aus einer Aluminiumfolie auf die Membran heiß aufgesiegelt, so dass auch das Füllloch verschlossen wurde und eine Abzugsecke zum Entfernen der Schutzfolie entstand.

### c) Erfindungsgemäße Vorrichtung:

Mit einer Verbundfolie, hergestellt aus einer Barex™-Folie (Dicke: 250 µm) und einer Hochdruck-Polyethylenfolie (Dicke: 50 µm), wurde ein Behälter gezogen. Auf den Behälter wurde eine Membran aus einer Verbundfolie, hergestellt aus einer Polyethylenfolie mit 19 Gew.-% Ethylacrylat (Dicke: 130 µm) und einer Hochdruck-Polyethylenfolie (Dicke: 20 µm), heiß so aufgesiegelt, dass zwischen Behälter und Membran ein Füllloch offen blieb. Nach dem Befüllen des Behälters mit der Wirkstoffzusammensetzung wurde eine Schutzfolie, bestehend aus einer Polyvinylalkoholfolie (Dicke: 10 bis 500 µm) auf die Membran heiß aufgesiegelt, so dass auch das Füllloch verschlossen wurde und eine Abzugsecke zum Entfernen der Schutzfolie entstand.

Das Wirkstoffabgabevermögen der drei Vorrichtungen wurde über einen Zeitraum von 47 Tagen untersucht. Die Ergebnisse zeigen deutlich, dass die erfindungsgemäße Vorrichtung eine höhere Diffusionsrate des in der Wirkstoffzusammensetzung enthaltenen Wirkstoffs ermöglicht (vgl. Fig. 4).

### Beispiel 6: Verschiedene Wirkstoffzusammensetzungen

### a) Wirkstoffzusammensetzung gegen Bronchialbeschwerden, Kopfschmerzen und Schlafstörungen:

2,50 g Dihydroterpineol, 2,50 g Menthol, 5,00 g Campher, 1,50 g Eucalypthus, 0,75 g Kiefernadelöl, 1,00 g Anisöl, 1,00 g Fenchelöl, 0,60 g Dihydromyrcenol, 0,05 g Thymianöl, 5,00 g Lavendelöl, 0,10 g Arnikablütenöl, 0,50 g Kamillenöl, 79,10 g isoparaffinischer Kohlenwasserstoff und 1,00 g Baldrianöl.

### b) Wirkstoffzusammensetzung zur Desodorierung und/oder Beduftung von Katzentoiletten:

4,00 g Benzylacetat, 2,00 g Heliotropin, 1,00 g 6-Methylcumarin, 2,00 g Ethylvanillin, 1,00 g Hexylacetat, 90,00 g isoparaffinischer Kohlenwasserstoff.

### c) Wirkstoffzusammensetzung zur Abwehr und/oder Bekämpfung von Insekten:

10 Gew.-% Lavendelöl, 33 Gew.-% Lavandinöl, 22 Gew.-% Linalylacetat, 5 Gew.-% Isononylacetat und 30 Gew-% isoparaffinischer Kohlenwasserstoff.

## Patentansprüche

1. Vorrichtung zur Freisetzung wenigstens eines Wirkstoffs, umfassend
(i) einen Behälter (1) aus einem ersten Hüllmaterial, wobei der Behälter (1) eine Aufnahmekammer (2) zur Aufnahme einer den wenigstens einen Wirkstoff umfassenden Zusammensetzung bildet und an einer offenen Seite einen am Rand der Kammer umlaufenden Flansch (3) aufweist,
(ii) eine Membran (4) aus einem zweiten Hüllmaterial umfassend eine Polyethylenfolie mit einem Gehalt von 5 bis 50 Gew.-% Ethylacrylat und/oder 5 bis 50 Gew.-% Ethylenvinylacetat, bezogen auf das Gesamtgewicht des Polyethylens, wobei die Membran (4) an dem Flansch (3) des Behälters (1) fest angebracht ist, und
(iii) eine Schutzfolie (5) aus einem dritten Hüllmaterial umfassend eine Polyvinylalkoholfolie, wobei die Schutzfolie (5) auf der Membran (4) angebracht ist und mit der Hand ergriffen und durch Ziehen mit der Hand von der Membran (4) entfernt werden kann.

2. Vorrichtung nach Anspruch 1, **dadurch gekennzeichnet, dass** das erste Hüllmaterial ausgewählt ist aus einer Polyacrylnitrilfolie, einer Polyesterfolie, einer Polypropylenfolie, einer Polyethylenfolie und einer eine Polyacrylnitrilfolie, eine Polyesterfolie, eine Polypropylenfolie und/oder eine Polyethylenfolie umfassenden Verbundfolie.

3. Vorrichtung nach Anspruch 2, **dadurch gekennzeichnet, dass** die Polyacrylnitrilfolie eine Dicke von 10 bis 600 µm aufweist.

4. Vorrichtung nach Anspruch 2 oder 3, **dadurch gekennzeichnet, dass** die Polyacrylnitrilfolie eine Barex™-Folie ist.

5. Vorrichtung nach Anspruch 2, **dadurch gekennzeichnet, dass** die Polypropylenfolie eine Dicke von 10 bis 600 µm aufweist.

6. Vorrichtung nach Anspruch 2, **dadurch gekennzeichnet, dass** die Polyethylenfolie eine Dicke von 10 bis 300 µm aufweist.

7. Vorrichtung nach Anspruch 2 oder 6, **dadurch gekennzeichnet, dass** die Polyethylenfolie eine Hochdruck-Polyethylenfolie oder eine Polyethylenfolie mit 5 bis 50 Gew.-% Ethylacrylat und/oder 5 bis 50 Gew.-% Ethylenvinylacetat, bezogen auf das Gesamtgewicht des Polyethylens, ist.

8. Vorrichtung nach Anspruch 7, **dadurch gekennzeichnet, dass** die Polyethylenfolie mit 5 bis 50 Gew.-% Ethylacrylat und/oder 5 bis 50 Gew.-% Ethylenvinylacetat, bezogen auf das Gesamtgewicht des Polyethylens, eine Dicke von 10 bis 100 µm aufweist.

9. Vorrichtung nach einem der Ansprüche 2 bis 8, **dadurch gekennzeichnet, dass** die Verbundfolie eine aus einer Polyacrylnitrilfolie, einer Hochdruck-Polyethylenfolie und einer Polyethylenfolie mit 5 bis 50 Gew.-% Ethylacrylat und/oder 5 bis 50 Gew.-% Ethylenvinylacetat, bezogen auf das Gesamtgewicht des Polyethylens, erzeugte Verbundfolie oder eine aus einer Polypropylenfolie, einer Hochdruck-Polyethylenfolie und einer Polyethylenfolie mit 5 bis 50 Gew.-% Ethylacrylat und/oder 5 bis 50 Gew.-% Ethylenvinylacetat, bezogen auf das Gesamtgewicht des Polyethylens, erzeugte Verbundfolie ist.

10. Vorrichtung nach einem der Ansprüche 1 bis 9, **dadurch gekennzeichnet, dass** das zweite Hüllmaterial ausgewählt ist aus einer Polyethylenfolie mit 5 bis 50 Gew.-% Ethylacrylat und/oder 5 bis 50 Gew.-% Ethylenvinylacetat, bezogen auf das Gesamtgewicht des Polyethylens, und einer eine Polyethylenfolie mit 5 bis 50 Gew.-% Ethylacrylat und/oder 5 bis 50 Gew.-% Ethylenvinylacetat, bezogen auf das Gesamtgewicht des Polyethylens, umfassenden Verbundfolie.

11. Vorrichtung nach Anspruch 10, **dadurch gekennzeichnet, dass** die Polyethylenfolie mit 5 bis 50 Gew.-% Ethylacrylat und/oder 5 bis 50 Gew.-% Ethylenvinylacetat, bezogen auf das Gesamtgewicht des Polyethylens, eine Dicke von 10 bis 100 µm aufweist.

12. Vorrichtung nach Anspruch 10 oder 11, **dadurch gekennzeichnet, dass** die Verbundfolie eine aus einer Polyethylenfolie mit 5 bis 50 Gew.-% Ethylacrylat und/oder 5 bis 50 Gew.-% Ethylenvinylacetat, bezogen auf das Gesamtgewicht des Polyethylens, und einer Hochdruck-Polyethylenfolie erzeugte Verbundfolie ist.

13. Vorrichtung nach Anspruch 12, **dadurch gekennzeichnet, dass** die Hochdruck-Polyethylenfolie eine Dicke von 10 bis 300 µm aufweist.

14. Vorrichtung nach einem der Ansprüche 1 bis 13, **dadurch gekennzeichnet, dass** das dritte Hüllmaterial ausgewählt ist aus einer Polyvinylalkoholfolie und einer eine Polyvinylalkoholfolie umfassenden Verbundfolie.

15. Vorrichtung nach Anspruch 14, **dadurch gekennzeichnet, dass** die Polyvinylalkoholfolie eine Dicke von 10 bis 500 µm aufweist.

16. Vorrichtung nach einem der Ansprüche 1 bis 15, **dadurch gekennzeichnet, dass** das erste Hüllmaterial eine aus einer Barex™-Folie, einer Hochdruck-Polyethylenfolie und einer Polyethylenfolie mit 5 bis 50 Gew.-% Ethylacrylat und/oder 5 bis 50 Gew.-% Ethylenvinylacetat, bezogen auf das Gesamtgewicht des Polyethylens, erzeugte Verbundfolie oder eine aus einer Polypropylenfolie, einer Hochdruck-Polyethylenfolie und einer Polyethylenfolie mit 5 bis 50 Gew.-% Ethylacrylat und/oder 5 bis 50 Gew.-% Ethylenvinylacetat, bezogen auf das Gesamtgewicht des Polyethylens, erzeugte Verbundfolie ist, das zweite Hüllmaterial eine aus einer Polyethylenfolie mit 5 bis 50 Gew.-% Ethylacrylat und/oder 5 bis 50 Gew.-% Ethylenvinylacetat, bezogen auf das Gesamtgewicht des Polyethylens, und einer Hochdruck-Polyethylenfolie erzeugte Verbundfolie ist und das dritte Hüllmaterial eine Polyvinylalkoholfolie ist.

17. Vorrichtung nach einem der Ansprüche 1 bis 16, **dadurch gekennzeichnet, dass** die Wandung des Behälters (1) eine Gesamtdicke von 10 bis 800 µm aufweist.

18. Vorrichtung nach einem der Ansprüche 1 bis 17, **dadurch gekennzeichnet, dass** die Membran (4) eine Gesamtdicke von 10 bis 400 µm aufweist.

19. Vorrichtung nach einem der Ansprüche 1 bis 18, **dadurch gekennzeichnet, dass** die Schutzfolie (5) eine Gesamtdicke von 10 bis 500 µm aufweist.

20. Vorrichtung nach einem der Ansprüche 1 bis 19, **dadurch gekennzeichnet, dass** die Membran (4) durch Versiegeln an dem Flansch (3) des Behälters (1) angebracht ist.

21. Vorrichtung nach einem der Ansprüche 1 bis 20, **dadurch gekennzeichnet, dass** die Schutzfolie (5) durch Versiegeln auf der Membran (4) angebracht ist.

22. Vorrichtung nach Anspruch 21, **dadurch gekennzeichnet, dass** die Schutzfolie (5) in dem dem Bereich des Flansches (3) des Behälters (1) entsprechenden Bereich und/oder in dem innerhalb des Bereichs des Flansches (3) des Behälters (1) liegenden Bereich auf der Membran (4) angebracht ist.

23. Vorrichtung nach einem der Ansprüche 1 bis 22, **dadurch gekennzeichnet, dass** die Vorrichtung eine Aufhängeeinrichtung umfasst.

24. Vorrichtung nach einem der Ansprüche 1 bis 23, **dadurch gekennzeichnet, dass** in der Aufnahmekammer (2) eine den wenigstens einen Wirkstoff umfassende Zusammensetzung enthalten ist.

25. Vorrichtung nach Anspruch 24, **dadurch gekennzeichnet, dass** der wenigstes eine Wirkstoff ausgewählt ist Duft- und Riechstoffen und beliebigen Kombinationen davon.

26. Vorrichtung nach Anspruch 25, **dadurch gekennzeichnet, dass** die Duft- und Riechstoffe natürliche und synthetische Duft- und Riechstoffe umfassen.

27. Vorrichtung nach einem der Ansprüche 24 bis 26, **dadurch gekennzeichnet, dass** die Zusammensetzung zusätzlich wenigstens eine Verbindung ausgewählt aus Lösungsmitteln, Verdampfungsmitteln, Farbstoffen und Konservierungsmitteln umfasst.

28. Verfahren zur Herstellung einer Vorrichtung nach einem der Ansprüche 1 bis 27, umfassend
(a) das Erzeugen des Behälters (1) durch Tiefziehen des ersten Hüllmaterials unter Ausbildung der Aufnahmekammer (2),
(b) das Erzeugen der Membran (4) durch Versiegeln der Aufnahmekammer (2) mit dem zweiten Hüllmaterial, wobei das Versiegeln im Bereich des Flansches (3) des Behälters (1) erfolgt und wobei eine kleine Stelle in diesem Bereich zum Befüllen der Aufnahmekammer (2) mit einer Zusammensetzung umfassend den wenigstens einen Wirkstoff offen bleibt,
(c) das Befüllen der Aufnahmekammer (2) mit der Zusammensetzung umfassend den wenigstes einen Wirkstoff, und
(d) das Erzeugen der Schutzfolie (5) durch Versiegeln oder Laminieren der Membran (4) mit dem dritten Hüllmaterial, wobei das Versiegeln in dem dem Bereich des Flansches (3) des Behälters (1) entsprechenden Bereich der Membran (4) und/oder in dem innerhalb dieses Bereiches liegenden Bereich der Membran (4) erfolgt.

29. Verfahren zur Herstellung einer Vorrichtung nach einem der Ansprüche 1 bis 27, umfassend
(a) das Erzeugen des Behälters (1) durch Tiefziehen des ersten Hüllmaterials unter Ausbildung der Aufnahmekammer (2),
(c) das Befüllen der Aufnahmekammer (2) mit der Zusammensetzung umfassend den wenigstes einen Wirkstoff, und
(b) das Erzeugen der Membran (4) durch Versiegeln oder Laminieren der Aufnahmekammer (2) mit dem zweiten Hüllmaterial, wobei das Versiegeln im Bereich des Flansches (3) des Behälters (1) erfolgt,
(d) das Erzeugen der Schutzfolie (5) durch Versiegeln der Membran (4) mit dem dritten Hüllmaterial, wobei das Versiegeln in dem dem Bereich des Flansches (3) des Behälters (1) entsprechenden Bereich der Membran (4) und/oder in dem innerhalb dieses Bereiches liegenden Bereich der Membran (4) erfolgt.

30. Verwendung einer Vorrichtung nach einem der Ansprüche 1 bis 28 zur Desodorierung und/oder Beduftung von Räumen.

31. Verwendung nach Anspruch 30, **dadurch gekennzeichnet, dass** die Räume ausgewählt sind aus Gebäudeinnenräumen, Fahrzeuginnenräumen, Geschirrspülmaschineninnenräumen, Waschmaschineninnenräumen, Kühlschrankinnenräumen, Mülleimerinnenräumen, Innenräumen von Tiertoiletten und Innenräumen von Einrichtungsgegenständen.

32. Verwendung einer Vorrichtung nach einem der Ansprüche 1 bis 28 zur Abwehr und/oder Bekämpfung von Insekten.

33. Vorrichtung nach einem der Ansprüche 1 bis 28 zur Behandlung von Erkrankungen des Atemwegssystems.

34. Vorrichtung nach einem der Ansprüche 1 bis 28 zur Behandlung von Kopfschmerzen.

35. Vorrichtung nach einem der Ansprüche 1 bis 28 zur Behandlung von Schlafstörungen.
